# FASCICULE DE BREVET EUROPEEN

(11) **EP 2 131 794 B1**
(45) Date de publication et mention de la délivrance du brevet: **04.09.2019**
(21) Numéro de dépôt: 08799822.5
(22) Date de dépôt: 14.03.2008
(51) Int. Cl.: A61F 2/52, A61G 7/057, A61L 27/44, C08K 3/34, C08K 5/15, C08L 83/04

(54) **ARTICLE COMPRENANT UN GEL SILICONE ADDITIVE D'UN PRINCIPE ACTIF ANTI-ODEUR**
AUS EINEM SILICIUMGEL MIT EINEM GERUCHSUNTERDRÜCKENDEN WIRKSTOFF ZUSAMMENGESETZTER ARTIKEL
ITEM COMPOSED OF A SILICON GEL CONTAINING AN ODOR MASKING ACTIVE INGREDIENT

(30) Priorité: 15.03.2007 FR 0753858
(43) Date de publication de la demande: 16.12.2009
(73) Titulaire: ELKEM SILICONES France SAS, 69003 Lyon (FR); Bluestar Silicones Germany, 23566 Lübeck (DE)
(72) Inventeur: MARTIN, François, 23552 Luebeck (DE); APPELT, Mathias, 23564 Luebeck (DE)
(74) Mandataire: Mekki, Boualem
(86) Numéro de dépôt international: PCT/FR2008/000333
(87) Numéro de publication internationale: WO 2008/129171

(56) Documents cités:
- FR-A- 2 770 220
- US-A- 5 132 385
- US-A- 5 466 726
- US-A- 5 534 609
- US-A- 6 162 250
- US-B1- 6 235 861
- US-B1- 6 420 037

## Description

La présente invention a pour objet un article, tel qu'une prothèse mammaire externe, un coussin anti-escarres ou un matelas anti-escarres, comprenant une enveloppe fermée **A** constitué d'un matériau souple **B** contenant comme matériau de remplissage un gel silicone comprenant un principe actif anti-odeur **C.**

Les gels de silicone et autres gels sont couramment employés dans le domaine médical que ce soit à usage externe (prothèses mammaires, coussins ou matelas médicaux) ou à usage interne (prothèses mammaires placées in situ). Ils sont d'une très grande mobilité et ont de très bonnes propriétés mécaniques, leur densité étant proche de celle des tissus humains.

En effet, il existe un nombre relativement important de types de prothèses mammaires externes destinées à être placées dans un bonnet d'un soutien-gorge. Les premières prothèses mammaires étaient en polyuréthane moulé de densité légère. De même que les prothèses mammaires en plastique gonflable qui ont été mises au point ultérieurement, elles présentaient l'inconvénient de ne pas posséder la même mobilité qu'un véritable sein, d'être plus légères, ce qui créait chez la femme un déséquilibre, et de tenir difficilement en place.

Des prothèses mammaires en silicone moulé qui ont la forme d'un sein sont connues. Néanmoins, elles n'en respectent pas exactement le poids, et n'en possèdent pas les mouvements naturels.

A présent, de nombreux modèles de prothèses mammaires externes contiennent une enveloppe souple formée à partir d'un film polyuréthane et remplit d'un gel silicone.

Un autre domaine où les gels silicones sont fréquemment utilisés est celui de la prévention des plaies de pression.

On sait que les personnes astreintes à rester longtemps en position assise ou allongée sont exposés au risque de formation de plaies de pression résultant d'une compression prolongée des tissus qui empêche le sang de circuler, ce qui entraîne une nécrose puis une décomposition des tissus qui s'infectent.

Les plaies de pression constituent un problème majeur pour les personnes dont la mobilité est réduite (utilisateurs de fauteuil roulant, personnes âgées, personnes alitées, etc.) ou dont certaines surfaces tissulaires sont dépourvues de sensation. Les plaies de pression sont des ulcérations (nécrose ischémique cutanée et sous-cutanée) consécutives à l'appui prolongé et continuel d'un relief osseux sur un plan dur. Désignées également par les termes « plaie », « ulcère » ou « escarre », elles apparaissent généralement dans les régions du corps présentant une proéminence osseuse près de la peau. Les coussins ou matelas médicaux constituent encore aujourd'hui la méthode de prévention la plus efficace.

On connaît déjà des coussins et des matelas anti-escarres constitués par des poches gonflables ou par des poches remplies d'un liquide ou d'un gel ou encore des coussins ou matelas en un matériau cellulaire.

Cependant, lorsque les gels de silicone sont utilisés comme matériau de remplissage de poches ou enveloppes en matériau souple, en particulier pour des enveloppes du type film plastique à base de polyuréthane ou du type élastomère silicone, pour la préparation d'article tel qu'une prothèse mammaire externe, un coussin ou un matelas médical, ils présentent l'inconvénient de dégager une odeur désagréable lorsque le gel silicone est vulcanisé lors de la préparation et de la mise en forme de l'article par moulage ou tout autre technique connue de l'homme du métier. En effet pour réaliser ces articles, on introduit une composition silicone précurseur d'un gel silicone dans une poche ou enveloppe en matériau souple et on fait polymériser le matériau de remplissage dans un moule par une élévation de température ou vulcanisation. L'interaction entre le gel silicone et l'enveloppe, en particulier lorsqu'elle est du type film polyuréthane, associée à une élévation de température lors de la vulcanisation semble générer des produits de dégradation responsable de l'apparition d'odeurs nauséabondes.

Dans cet état de connaissance, l'un des objectifs essentiel de la présente invention est de fournir un article comprenant une enveloppe fermée constitué d'un matériau souple du type film plastique à base de polyuréthane ou du type élastomère silicone et contenant un gel silicone comme matériau de remplissage et qui ne présente pas les problèmes d'odeur mentionnés ci-dessus.

La Demanderesse a mis en oeuvre d'importants moyens de recherche et de nombreuses expérimentations pour atteindre cet objectif parmi d'autres et au terme de cela, elle a eu le mérite de trouver, de manière tout à fait surprenante et inattendue, qu'il convient d'introduire dans une composition silicone standard, apte à réticuler pour former un gel silicone, un aluminosilicate pour supprimer les problèmes d'odeur dans les applications mentionnées ci-dessus.

La présente invention a pour objet un article comprenant une enveloppe fermée **A** constitué d'un matériau souple de préférence du type film plastique à base de polyuréthane ou du type élastomère silicone, ladite enveloppe fermée **A** contenant comme matériau de remplissage un gel silicone obtenu par réticulation d'une composition silicone **B** réticulable en gel par hydrosilylation caractérisé en ce que ladite composition silicone **B** comprend au moins un principe actif anti-odeur **C** qui est un aluminosilicate.

Selon les caractéristiques essentielles de l'invention l'article comprenant une enveloppe fermée **A** constitué d'un matériau souple de préférence du type film plastique à base de polyuréthane ou du type élastomère silicone matelas en question se caractérise essentiellement en ce qu'il contient comme matériau de remplissage un gel silicone comprenant un principe actif anti-odeur **C** qui est un aluminosilicate.

Selon un mode de réalisation avantageux le principe actif anti-odeur **C** est un aluminosilicate, de préférence une zéolithe et encore plus préférentiellement une zéolithe synthétique.

Les zéolithes sont des matériaux poreux cristallins. Structurellement, une zéolithe est un assemblage de cages d'aluminosilicates cristallisées. La cage unitaire consiste en un assemblage de tétraèdres associant des complexes d'oxyde d'aluminium et d'oxydes de silicium, et partageant les atomes d'oxygène. Une zéolithe est donc caractérisée par un assemblage de tétraèdres QO₄, où Q représente en général les atomes Si et Al, mais également Ti, Ge, B, Fe et Ga. Les charges anioniques sont équilibrées par la présence de cations alcalins ou alcalino-terreux (Na, K, Li, Ca) et s'organisent finalement selon la formule M_{x/n}[AlₓSi_{y}O_{2(x+y)}], zH2O. Selon la valeur du rapport y/x, on peut classer les structures en plusieurs types. Plus de 120 types de structures élémentaires ont été trouvées, classées selon un code à trois lettres par l'International Zeolite Association. Une signification de ces codes est par exemple décrite dans le recueil de structures zéolithiques: Ch. Baerlocher, W.W.Meier, D.H. Oison, Atlas of zeolite framework types, Fifth revised Edition, Elsevier, Amsterdam 2001, 3-18.

Des synthèses de zéolithes sont décrites par exemple dans les publications H. Kessler, Synthesis of Molecular Sieves, Comprehensive Supramolecular Chemistry, G. Alberti, T. Bein (Eds.), Vol. 7 Pergamon, Oxford, 1996, 425-464, et C.S. Cundy et P.A. Cox, The Hydrothermal Synthesis of Zeolites: History and Development from the Earliest Days to the Present Time, Chem. Rev. 2003, 103, 663-701.

De préférence, le composé comprend entre 4 et 20, de préférence entre 4 et 12, mieux 6, 7, 8, 9, 10 ou 12 atomes dudit élément des colonnes 3 à 13.

Les gels silicones sont traditionnellement obtenus par réticulation d'une composition silicone comprenant :
- au moins un polyorganosiloxane (A) ayant en moyenne deux groupes alcényle liés à du silicium par molécule, lesdits groupes alcényle comptant chacun 2 à 6 atomes de carbone et aucun atome de silicium n'étant lié à plus d'un seul groupe alcényle,
- au moins un composé hydrogéné du silicium (B) ayant au moins 2 et de préférence au moins trois atomes d'hydrogène liés à du silicium par molécule,
- éventuellement au moins un polyorganosiloxane non fonctionnalisé (C), et
- un catalyseur d'hydrosylilation (D) à base de platine.

Pour toutes ces applications, les propriétés physiques de ces gels sont adaptées suivant l'utilisation en faisant varier les taux de motifs siloxyles porteurs de fonctions vinyle et SiH.

En général, le polydiorganosiloxane (A) contient en moyenne deux groupes alcényle liés à du silicium par molécule, chaque groupe alcényle étant lié à un atome de silicium différent. Le polydiorganosiloxane (A) est un polymère sensiblement linéaire, bien qu'un faible degré de ramification puisse exister. De préférence, les groupes alcényle sont fixés à des atomes de silicium qui sont éloignés entre eux dans la molécule, et au mieux, ils sont fixés aux atomes de silicium terminaux de la chaîne siloxane. Les groupes alcényle comptent au maximum 6 atomes de carbone et il peut s'agir par exemple de groupes vinyle, allyle ou hexényle, bien que ce soient de préférence des groupes vinyle. Les substituants organiques restants du polydiorganosiloxane (A) sont choisis parmi les groupes alkyle et aryle, s'agissant de préférence de groupes alkyle n'ayant pas plus de 8 atomes de carbone et de groupes phényle. Des exemples de ces substituants restants sont les groupes méthyle, éthyle, propyle, isobutyle et phényle. Les plus volontiers mis en oeuvre sont des polydiméthylsiloxanes α,ω (diméthyvinylsiloxy) ou des polyorganosiloxanes de type poly(diméthylsiloxy) (méthylvinylsiloxy) α,ω (diméthylvinylsiloxy).

Le polydiorganosiloxane (A) est un produit commercial comme par exemple les produits de la gamme des RHODORSIL® 621V de la société Bluestar Silicones, et sont largement divulgués tant en ce qui concerne leurs structures que leurs synthèses dans la littérature technique.

D'une manière préférée, le polydiorganosiloxane (A) est sensiblement linéaire et possède une viscosité dynamique inférieure ou égale à 200 000 m.Pa.s, de préférence à 170000 mPa.s et encore plus préférentiellement comprise entre 20 et 165000 mPa.s.

Selon une autre variante, le % en poids de groupes réactifs alcényles liés directement à un atome de silicium, est compris entre 0,025 % et 3%.

Le composé hydrogéné du silicium (B) est en général un polyorganosiloxane, ou un silane, comportant au moins 2, de préférence 3, atomes d'hydrogène liés à du silicium par molécule. Ces atomes d'hydrogène peuvent être situés sur des motifs siloxanes terminaux et également sur des motifs siloxanes se trouvant dans la chaîne polymère, ou bien ils peuvent être situés uniquement à l'intérieur de la chaîne siloxane.

En pratique, les polyorganohydrogénosiloxanes (B) mis en oeuvre sont par exemple les polyorganosiloxanes de type poly(diméthylsiloxy)-(siloxyméthylhydrogéno)- α,ω-(diméthylhydrogénosiloxy) et les polydiméthylsiloxanes -α,ω-(diméthylhydrogénosiloxy). Ces POS (I) sont des produits commerciaux et sont largement divulgués tant en ce qui concerne leurs structures que leurs synthèses dans la littérature technique.

Pour les polyorganosiloxanes non fonctionnalisé (C), les plus volontiers mis en oeuvre sont des huiles polydimethylsiloxanes-α,ω-(triméthylsiloxy) ou PDMS. Ces polyorganosiloxanes sont des produits commerciaux comme par exemple les produits de la gamme des RHODORSIL® 47V (par exemple 47V50, 47V100, 47V500, 47V500, 47V12500 ou 47V30000) de la société Bluestar Silicones, et sont largement divulgués tant en ce qui concerne leurs structures que leurs synthèses dans la littérature technique.

D'une manière préférée, le polyorganosiloxanes non fonctionnalisé (C) est sensiblement linéaire et possède une viscosité dynamique inférieure ou égale à 50 000 mPa.s, de préférence comprise entre 20 et 40 000 mPa.s.

Le catalyseur (D) est un autre élément important de la composition selon l'invention. Il s'agit, de préférence, d'un complexe organométallique du platine ou bien encore de l'un des catalyseurs à base de platine traditionnellement mis en oeuvre pour la catalyse de réactions d'hydrosilylation entre par exemple, des groupes ≡SiH et des groupes ≡Si-Vinyle. A titre d'exemples, on peut citer le platine noir, l'acide chloroplatinique, un acide chloroplatinique modifié par un alcool, un complexe de l'acide chloroplatinique avec une oléfine, un aldéhyde, un vinylsiloxane ou un alcool acétylénique, entre autres. Le brevet US N° 2 823 218 divulgue un catalyseur d'hydrosilylation du type acide chloroplatinique et le brevet US N°3 419 593 est relatif à des catalyseurs formés par des complexes d'acide chloroplatinique et d'organosilicone du type vinylsiloxane. Des complexes de platine et d'hydrocarbures utiles comme catalyseur d'hydrosilylation sont divulgués par les brevets US N° 3 159 601 et 3 159 662. Le brevet US N ° 3 723 497 décrit un acétylacétonate de platine et le brevet US N° 3 220 972 a pour objet des catalyseurs à base d'alcoolate de platine.

Pour le composant (D), on entend par quantité efficace d'au moins un catalyseur de réaction d'hydrosilylation, la quantité suffisante pour amorcer la réaction d'hydrosilylation. Concernant la quantité catalytiquement efficace à mettre en oeuvre, il va de soi que l'homme du métier du domaine considéré est parfaitement à même de déterminer la quantité optimale de catalyseur pour promouvoir la réaction d'hydrosilylation . Cette quantité dépend notamment de la nature du catalyseur et des POS en cause. Pour fixer les idées on peut indiquer qu'elle sera comprise entre 0,001 et 0,5 % en poids par rapport au poids total de la composition.

De préférence, la quantité des constituants (A), (B), (C) et (D) est choisie de manière à ce que le rapport molaire r des atomes d'hydrogène liés au silicium aux radicaux alcényles (X) liés au silicium soit compris entre 0,5 :1 et 5 :1.

La composition silicone selon l'invention peut en outre comprendre au moins un ralentisseur de la réaction d'addition ou un inhibiteur de réticulation choisi parmi les composés suivants :
- polyorganosiloxanes substitués par au moins un alcényle pouvant se présenter éventuellement sous forme cyclique, le tétraméthylvinyltétrasiloxane étant particulièrement préféré,
- la pyridine,
- les phosphines et les phosphites organiques,
- les amides insaturés,
- les maléates alkylés, et
- les alcools acétyléniques.

Ces alcools acétyléniques, (voir FR-A-1 528 464 et FR-A-2 372 874), qui font partie des bloqueurs thermiques de réaction d'hydrosilylation préférés, ont pour formule :

R' -(R")C(OH)-C≡CH

formule dans laquelle,
- R' est un radical alkyle linéaire ou ramifié, ou un radical phényle ;
- R" est H ou un radical alkyle linéaire ou ramifié, ou un radical phényle ; les radicaux R', R" et l'atome de carbone situé en α de la triple liaison pouvant éventuellement former un cycle ; et
- le nombre total d'atomes de carbone contenu dans R' et R" étant d'au moins 5, de préférence de 9 à 20.

Lesdits alcools sont, de préférence, choisis parmi ceux présentant un point d'ébullition supérieur à 250°C. On peut citer à titre d'exemples :
- l'éthynyl-1-cyclohexanol-1 ;
- le méthyl-3 dodécyne-1 ol-3 ;
- le triméthyl-3,7,11 dodécyne-1 ol-3 ;
- le diphényl-1,1 propyne-2 ol-1 ;
- l'éthyl-3 éthyl-6 nonyne-1 ol-3 ;
- le méthyl-2 butyne-3 ol-2 ;
- le méthyl-3 pentadécyne-1 ol-3.

Ces alcools α-acétyléniques sont des produits du commerce. Un tel ralentisseur est présent à raison de 3 000 ppm au maximum, de préférence à raison de 100 à 1000 ppm par rapport au poids total des polyorganosiloxanes de la composition silicone.

De manière connue en soi, la composition élastomère silicone peut encore être additionnée de divers additifs classiques comme par exemple des charges ou des colorants.

Pour améliorer la stabilité au stockage de la composition selon l'invention et pour fournir aux utilisateurs une forme commerciale aisément manipulable, il est prévu un système à au moins deux composants A et B comprenant les constituants (A), (B), (C) et éventuellement le constituant (E) de la composition silicone réticulable en gel adhésif par hydrosilylation telle que définie selon l'invention, avec comme condition que le catalyseur de réaction d'hydrosilylation (D) soit séparé du constituant (B).

Pour simplifier l'utilisation, il est préférable de proposer un système bi-composant dont les proportions A : B sont comprises entre 10 :100 et 100 :10 et de préférence entre 40 : 60 et 60 : 40, et encore plus préférentiellement de 50 : 50 parties en poids environ.

S'agissant de la préparation du gel, on peut préciser que la réticulation de la composition en gel intervient à la température ambiante ou après chauffage à des températures comprises entre 50 et 200° C par exemple. Dans ce contexte, les durées de réticulation nécessaires sont, par exemple, comprises entre quelques mn et 1 heure 30 mn. Le gel adhésif réticulé obtenu à partir de la composition décrite ci-dessus forme un objet à part entière de la présente invention.

Selon un mode de réalisation préféré la composition silicone **B** réticulable en gel par hydrosilylation comprend jusqu'à 1 %, de préférence jusqu'à 0, 5 % et encore plus préférentiellement entre 0,01 et 0,1% en poids, par rapport au poids total de ladite composition silicone **B**, dudit principe actif anti-odeur **C**.

Selon un mode de réalisation préféré de l'invention l'article selon l'invention est une prothèse mammaire externe, un coussin anti-escarres ou un matelas anti-escarres.

Ces produits sont largement diffusés et décrits dans le commerce et sont bien connus de l'homme du métier.

L'invention concerne aussi :
- l'utilisation d'au moins un aluminosilicate comme principe actif anti-odeur **C** dans les gels silicones obtenus par réticulation d'une composition silicone **B** réticulable en gel par hydrosilylation et destinés aux prothèses de reconstitution plastique ou coussins à usage orthopédique et autre,
- l'utilisation d'au moins un aluminosilicate, une zéolithe étant particulièrement préférée et une zéolithe synthétique encore plus préférée, comme principe actif anti-odeur **C** dans les gels silicones obtenus par réticulation d'une composition silicone **B** réticulable en gel par hydrosilylation et destinés aux prothèses mammaires externes, aux coussins anti-escarres ou aux matelas anti-escarres, et
- l'utilisation caractérisée en ce que l'aluminosilicate est une zéolithe et de préférence une zéolithe synthétique.

Les exemples non limitatifs qui suivent, montrent diverses possibilités de formulation des compositions selon l'invention ainsi que les caractéristiques et les propriétés des gels silicones obtenus par réticulation desdites compositions.

### EXEMPLES:

### 1) La liste ci-dessous décrit les matières premières utilisées dans les compositions des parties A et B de ce gel :

- Huile (A) (SiVi) = huile polydiméthylsiloxane α,ω (diméthylvinylsiloxy) de viscosité 100 000 mPa.s,
- Huile (B) (SiH) = huile poly(diméthylsiloxy)(siloxyméthylhydrogéno)-α,ω-(diméthylhydrogénosiloxy) de viscosité 25 mPa.s
- Huile (C) (PDMS) = huile polydiméthylsiloxane α,ω(triméthylsiloxy) de viscosité de 100 mPa.s,
- Empatage (D): mélange huile polydiméthylsiloxane à groupes vinyles terminaux + 30 % en poids silice de pyrogénation amorphe,
- Huile (E) (cyclique D^{Vi}) : Méthylvinylcyclosiloxane
- Mélange (F): 10% en poids d'une huile α,ω(triméthylsiloxy)-poly(siloxyméthylhydrogéno) (structure MD'₅₀M), dans une huile polydiméthylsiloxane de viscosité 50 mPa.s,
- Huile (G) (SiH) (huileα,ω(triméthylsiloxy poly(diméthylsiloxy)(siloxyméthylhydrogéno) viscosité de 10 mPa.s
- Catalyseur (F) = complexe organométallique du platine en solution utilisé comme catalyseur de réticulation ; les concentrations de ce catalyseur sont données en % en poids de Pt métal de degré d'oxydation = 0 par rapport à la masse totale de la composition.

### 2) Le Tableau 1 décrit les concentrations de chacun de ces constituants dans les parties A et B :

**Tableau 1 : Constitution des compositions testées :**

| Composition | partie A | partie B |
|---|---|---|
| Constituants | Concentration en poids (%) | |
| huile (C) (PDMS) | 86,62 | 84,82 |
| Huile (A) (SiVi) | 13,19 | 10,48 |
| Catalyseur (F) | 0,10 | / |
| Huile (E) (cyclique D^{Vi}) | 0,09 | 0,54 |
| Huile (B) (SiH) | / | 0,33 |
| Mélange (F) | / | 0,03 |
| Empatage (D) | / | 0,25 |
| Huile (G) (SiH) | / | 3,55 |
| Total | 100 | 100 |

La composition décrite se présente sous forme bicomposante et la réticulation s'effectue après mélange de deux parties nommées A et B dans un rapport 50 /50 à 25°C. On obtient ainsi un gel comparatif (C-1).

Un deuxième gel (I-1), selon l'invention, est préparé en mélangeant les parties A et B décrite ci-dessus (1 :1) auquel auquel on a ajouté 0,05% en poids par rapport au poids total de la composition une zéolithe ABSCENTS® 1000 fournie par la société UOP.

### Test pour évaluer le dégagement d'odeur :

A ces gels,(C-1) et (I-1), on incorpore des morceaux de 2cm² de surface d'un film de polyuréthane fourni par la société Pharetra (épaisseur du film : de 60 à 70 microns), puis on vulcanise l'ensemble à 120°C pendant 1h30. Les résultats des tests sont présentés dans le Tableau 2.

**Tableau 2 : Tests après vulcanisation.**

| | Présence d'odeurs nauséabondes (aminées) |
|---|---|
| Gel C-1 (comparatif) | OUI |
| Gel (I-1) Invention | NON |

Le gel selon l'invention (I-1) ne présente pas d'odeur nauséabonde (pas d'odeur aminée) alors que le gel selon l'exemple comparatif (C-1) présente une forte odeur neauséabonde.

Les mêmes résultats (absence d'odeur nauséabonde) ont été obtenus lors de l'utilisation du gel silicone selon l'invention comme matériau de remplissage d'enveloppe fermée constitué d'un matériau souple à base de polyuréthane dans la fabrication d'une prothèse mammaire externe, d'un coussin anti-escarres et d'un matelas anti-escarres.

## Revendications

1. Article comprenant une enveloppe fermée **A** constitué d'un matériau souple de préférence du type film plastique à base de polyuréthane ou du type élastomère silicone, ladite enveloppe fermée **A** contenant comme matériau de remplissage un gel silicone obtenu par réticulation d'une composition silicone **B** réticulable en gel par hydrosilylation **caractérisé en ce que** ladite composition silicone **B** comprend au moins un principe actif anti-odeur **C** qui est un aluminosilicate.

2. Article selon la revendication **1 caractérisé en ce qu'**il s'agit d'une prothèse mammaire externe, d'un coussin anti-escarres ou d'un matelas anti-escarres.

3. Article selon la revendication 1 **caractérisé en ce que** l'aluminosilicate est une zéolithe de préférence une zéolithe synthétique.

4. Article selon la revendication 1 **caractérisé en ce que** ladite composition silicone **B** réticulable en gel par hydrosilylation comprend jusqu'à 1 %, de préférence jusqu'à 0, 5 % et encore plus préférentiellement entre 0,01 et 0,1% en poids, par rapport au poids total de ladite composition silicone **B**, dudit principe actif anti-odeur **C.**

5. Utilisation d'au moins un aluminosilicate comme principe actif anti-odeur **C** dans les gels silicones obtenus par réticulation d'une composition silicone **B** réticulable en gel par hydrosilylation et destinés aux prothèses de reconstitution plastique ou coussins à usage orthopédique et autre.

6. Utilisation d'au moins un aluminosilicate comme principe actif anti-odeur **C** dans les gels silicones obtenus par réticulation d'une composition silicone **B** réticulable en gel par hydrosilylation et destinés aux prothèses mammaires externes, aux coussin anti-escarres ou aux matelas anti-escarres.

7. Utilisation selon la revendication 6 **caractérisée en ce que** l'aluminosilicate est une zéolithe et de préférence une zéolithe synthétique.

## Patentansprüche

1. Artikel, umfassend eine geschlossene Umhüllung **A** aus einem flexiblen Material, vorzugsweise vom Typ Kunststofffilm auf Polyurethan-Basis oder vom Silikonelastomer-Typ, wobei die geschlossene Umhüllung **A** als Füllstoff ein durch Vernetzung einer vernetzbaren Silikonzusammensetzung **B** zu einem Gel durch Hydrosilylierung erhaltenes Silikongel enthält, **dadurch gekennzeichnet, dass** die Silikonzusammensetzung **B** mindestens einen geruchsunterdrückenden Wirkstoff **C**, bei dem es sich um ein Aluminosilikat handelt, umfasst.

2. Artikel nach Anspruch 1, **dadurch gekennzeichnet, dass** es sich um eine externe Brustprothese, ein Kissen gegen Dekubitus oder eine Matratze gegen Dekubitus handelt.

3. Artikel nach Anspruch 1, **dadurch gekennzeichnet, dass** es sich bei dem Aluminosilikat um einen Zeolith und vorzugsweise einen synthetischen Zeolith handelt.

4. Artikel nach Anspruch 1, **dadurch gekennzeichnet, dass** die durch Hydrosilylierung zu einem Gel vernetzbare Silikonzusammensetzung B bis zu 1 Gew.-%, vorzugsweise bis zu 0,5 Gew.-% und noch weiter bevorzugt zwischen 0,01 und 0,1 Gew.-%, bezogen auf das Gesamtgewicht der Silikonzusammensetzung **B**, des geruchsunterdrückenden Wirkstoffs **C** umfasst.

5. Verwendung mindestens eines Aluminosilikats als geruchsunterdrückender Wirkstoff **C** in durch Vernetzung einer durch Hydrosilylierung zu einem Gel vernetzbaren Silikonzusammensetzung B erhaltenen und für Kunststoffrekonstitutionsprothesen oder Kissen für die orthopädische Verwendung und dergleichen bestimmten Silikongelen.

6. Verwendung mindestens eines Aluminosilikats als geruchsunterdrückender Wirkstoff **C** in durch Vernetzung einer durch Hydrosilylierung zu einem Gel vernetzbaren Silikonzusammensetzung **B** erhaltenen und für externe Brustprothesen, Kissen gegen Dekubitus oder Matratzen gegen Dekubitus bestimmten Silikongelen.

7. Verwendung nach Anspruch 6, **dadurch gekennzeichnet, dass** es sich bei dem Aluminosilikat um einen Zeolith und vorzugsweise einen synthetischen Zeolith handelt.

## Claims

1. Article comprising a closed envelope **A** formed from a supple material, preferably of the polyurethane-based plastic film type or of the silicone elastomer type, said closed envelope **A** containing as filling material a silicone gel obtained by crosslinking α silicone composition **B** that can be crosslinked into α gel by hydrosilylation, **characterized in that** said silicone composition **B** comprises at least one antiodor active principle **C** which is an aluminosilicate.

2. Article according to Claim 1, **characterized in that** it is an external mammary prosthesis, an anti-eschar cushion or an anti-eschar mattress.

3. Article according to Claim 1, **characterized in that** the aluminosilicate is a zeolite, preferably a synthetic zeolite.

4. Article according to Claim 1, **characterized in that** said silicone composition **B** that can be crosslinked into a gel by hydrosilylation comprises up to 1%, preferably up to 0.5% and even more preferentially between 0.01% and 0.1% by weight, relative to the total weight of said silicone composition **B**, of said antiodor active principle **C**.

5. Use of at least one aluminosilicate as antiodor active principle **C** in silicone gels obtained by crosslinking a silicone composition **B** that can be crosslinked into a gel by hydrosilylation and intended for plastic reconstitution prostheses or cushions for orthopaedic use, and the like.

6. Use of at least one aluminosilicate as antiodor active principle **C** in silicone gels obtained by crosslinking a silicone composition **B** that can be crosslinked into a gel by hydrosilylation and intended for external mammary prostheses, anti-eschar cushions or anti-eschar mattresses.

7. Use according to Claim 6, **characterized in that** the aluminosilicate is a zeolite and preferably a synthetic zeolite.
